(19) Europäisches Patentamt
European Patent Office
Office européen des brevets

(11) **EP 4 394 787 A1**

(12) **EUROPEAN PATENT APPLICATION**

(43) Date of publication:
**03.07.2024 Bulletin 2024/27**

(21) Application number: **23218926.6**

(22) Date of filing: **20.12.2023**

(51) International Patent Classification (IPC):
**G16H 20/17** (2018.01)   **G16H 50/20** (2018.01)
**G16H 50/50** (2018.01)   **A61M 5/142** (2006.01)
**A61M 5/172** (2006.01)

(52) Cooperative Patent Classification (CPC):
**G16H 20/17; G16H 50/20;** A61M 5/1723;
A61M 2005/14208; G16H 50/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.12.2022 FR 2214603**

(71) Applicant: **Diabeloop**
**38000 Grenoble (FR)**

(72) Inventors:
• **ROMERO-UGALDE, Hector**
**38420 LE VERSOUD (FR)**
• **LACHAL, Sylvain**
**38600 FONTAINE (FR)**

(74) Representative: **Pellegri, Michel Pascal Romain**
**Cabinet Vulpelex**
**52 Rue Montgolfier**
**69006 Lyon (FR)**

(54) **SYSTEM FOR ENSURING THE SAFETY OF A QUANTITY OF INSULIN TO BE INJECTED AND MEDICAL SYSTEM FOR REGULATING A GLYCEMIA OF A PERSON**

(57) A system for ensuring the safety of a quantity of insulin to be injected comprises:
- an insulin determination module (321) (322) configured to determine an initial quantity of insulin at a current instant based on:
*a value of glycemia; and/or
*an Insulin On Board (IOB) value;

- a safety module (323) configured to determine a quantity of insulin to be injected by:
°simulating an occurrence of a potential event having an impact on glycemia,

°determining a predicted value of glycemia corresponding to a value of glycemia determined for a future instant if the potential event had occurred and if the initial quantity of insulin had been injected at the current instant,
*performing a determination of whether the predicted value of glycemia is comprised within a predetermined range and

°if no, adjust the initial quantity of insulin to obtain a adjusted quantity of insulin,
°if yes, keep the initial quantity of insulin.

[Fig. 1]

**Description**

**FIELD OF THE INVENTION**

The present invention relates to a system for ensuring the safety of a quantity of insulin to be injected and to a medical system for regulating a glycemia of a person.

**BACKGROUND OF THE INVENTION**

In the field of healthcare, and more particularly in the field of diabetes, it is known to determine a concentration of blood glucose (i.e. glycemia) and to inject a quantity of insulin as a function of the determined concentration of blood glucose. Recently, so-called "closed-loop" systems have been developed, where the system is configured to evaluate a quantity to be injected, based on patient-related data and/or time-based data, such as past and/or present measures of glycemia, and to control the quantity of insulin to be injected based on this evaluation.

Other systems, so-called "semi closed-loop" systems have recently been developed, where the system is configured to determine a quantity of insulin to be injected in some special circumstances, such as meals, physical activity, etc. The quantity of insulin is subject to patient's approval and the patient needs to declare the special circumstances, which explain the name of "semi closed-loop" system.

For people with diabetes, it is particularly important to determine an accurate quantity of insulin to be injected since a too important amount could cause the person to go to a hypoglycemia state and a too little amount could cause the person to go to a hyperglycemia state. Generally, such quantity of insulin to be injected is a bolus of insulin, i.e. a one-time shot of insulin, Document US10980942 has developed a method that controls the amount of a bolus of insulin injected after a meal, to avoid any postprandial hypoglycemia.

The method comprises the announcement of a meal to be taken by a patient. The method then calculates an initial bolus amount and predicts a future glucose level of the patient in the postprandial time, based on the initial bolus amount, insulin delivery data and a current glucose measurement. If the predicted future glucose level is below a threshold, an adjusted bolus amount is determined, for which the predicted future glucose level is below a threshold. In other words, document US10980942 obtains an indication of a patient relatively to a meal and determines a safe amount of bolus to be injected according to the meal.

A first drawback of this document is that the adjusted bolus amount only relies on patient announcement. If the patient forgets to declare the meal, enters incorrect information about the meal and/or does not eat what he announced, then the adjusted bolus amount will be inaccurate.

A second drawback of this document is that it is only applicable on boluses, while generally a bolus is injected in addition with a basal.

The present disclosure aims to at least partially answer the above-presented technical problems.

**BRIEF SUMMARY OF THE INVENTION**

Thus, the invention relates to a system for ensuring the safety of a quantity of insulin to be injected, said system comprising:

- an insulin determination module, said insulin determination module being configured to determine an initial quantity of insulin at a current instant, said initial quantity of insulin being determined at least based on:

    *a value of glycemia; and/or
    *an Insulin On Board (IOB) value;

- a safety module configured to determine a quantity of insulin to be injected by:

    *performing a testing of the initial quantity of insulin by

        °simulating an occurrence of a potential event, wherein said potential event presents an impact on glycemia,
        °determining a predicted value of glycemia corresponding to a value of glycemia determined for a future instant if the potential event had occurred and if the initial quantity of insulin had been injected at the current instant,
    *performing a determination of whether the predicted value of glycemia is comprised within a predetermined range and
        °if no, adjust the initial quantity of insulin to obtain a adjusted quantity of insulin, said adjusted quantity of insulin being the quantity of insulin to be injected,
°if yes, keep the initial quantity of insulin, said initial quantity of insulin being the quantity of insulin to be injected.

The term "value of glycemia" includes a current, a past, an estimated, a predicted value of glycemia and/or an estimated glycemic trend.

The simulation of the occurrence of the potential event includes the simulation of the potential event happening at the current instant and/or the simulation of the potential event happening at a future instant.

The predetermined range is defined by a lower threshold and an upper threshold. If the predicted value of glycemia is inferior to the lower threshold, a risk of hypoglycemia may occur. If the predicted value of glycemia is superior to the upper threshold, a risk of hyperglycemia may occur.

By quantity of insulin, it is understood a bolus, a basal, a sequence of boluses and/or a sequence of basals.

The system allows to ensure the safety of a quantity of insulin that would be injected to a person. More exactly, the insulin determination module determines an initial quantity of insulin which is then analysed by the safety module before injecting the initial quantity of insulin. This can be particularly useful to avoid hypoglycemia or hyperglycemia.

More precisely, in the known document, at a current instant, a quantity of insulin determined by the system is supposed to have a positive impact on the glycemia of a patient, or at least to avoid any future hypoglycemia, and is then injected at the current instant. However, at a future instant, an event having a bad impact on glycemia, i.e. reducing the glycemia of a patient, can occur. At the future instant, it will be determined that no insulin is needed and that the glycemia of the patient is decreasing rapidly. Since the quantity of insulin has already been injected, it may be too late to just stop the injection of insulin at the future instant such that the person could go to hypoglycemia.

In this application, the safety module simulates the occurrence of such an event in a future instant and adjusts the quantity of insulin to be injected at the current instant accordingly. Hence, even if an event having an impact on glycemia will occur at the future instant, the quantity of insulin injected at the current instant won't be important enough to cause the person to undergo hypoglycemia or too little to cause the person to undergo hyperglycemia. In other words, it is ensured that after the injection of the quantity of insulin, the glycemia of the person won't be outside the predetermined range even if an unexpected event occurs.

According to different aspects, it is possible to provide the one and / or the other of the characteristics below taken alone or in combination.

According to an embodiment, the system further comprises a glycemia determination module, wherein:

- said glycemia determination module is configured to determine the value of glycemia, and wherein
- the insulin determination module is configured to determine the initial quantity of insulin if the determined value of glycemia satisfies a criterion.

In an embodiment the criterion is a hypoglycemia risk. If there is a risk of hypoglycemia, it is unlikely that the patient may need a dose of insulin. The glycemia determination module is then a first safety barrier to ensure that a quantity of insulin would not be delivered if there is no need, which could be dangerous.

Moreover, the glycemia determination module allows to reduce the calculation time and cost by indicating that the insulin determination module does not need to calculate the initial quantity of insulin when there is no need.

According to an embodiment, the safety module is configured to adjust the initial quantity of insulin by dividing or multiplying the initial quantity of insulin with a number greater than 1.

Alternatively, if the quantity of insulin is to be reduced, the initial quantity of insulin can be multiplied with a number between 0 and 1. Such a configuration allows to keep a quantity of insulin to be injected proportional to the initial quantity of insulin and therefore allows to obtain an accurate adjusted quantity of insulin to be injected and therefore increase the safety of a system's user.

The initial quantity of insulin is divided if the predicted value of glycemia is below the lower threshold. The initial quantity of insulin is multiplied if the predicted value of glycemia is above the upper threshold.

According to an embodiment, the safety module is configured to adjust the initial quantity of insulin by deducting a quantity of insulin from said initial quantity of insulin or by adding a quantity of insulin to said initial quantity of insulin.

According to an embodiment, the deducted quantity of insulin may be a function of the glycemia behaviour without any occurrence of the potential event and of the glycemia behaviour with the effective occurrence of the potential event. For example, in a normal case, i.e. with no occurrence of a potential event having an impact on glycemia, the insulin will naturally decrease according to a first delta. In the case where a potential event having an impact on glycemia effectively occurs, where the impact is a drop of the glycemia, the insulin will decrease faster than in the normal case according to a second delta. The deducted quantity of insulin can be computed as a function of the first and second deltas.

In the case where a potential event having an impact on glycemia effectively occurs, where the impact is a rise of the glycemia, the insulin will decrease according to a third delta. The added quantity of insulin can be computed as a function of the first and third deltas.

According to an embodiment, the safety module is configured to adjust the initial quantity of insulin by forcing the value of said initial quantity of insulin to 0.

Such a configuration allows to reduce the risk of hypoglycemia.

According to an embodiment, the safety module is further configured to iteratively adjust the initial quantity of insulin, to obtain the quantity of insulin to be injected, until the predicted value of glycemia is within the predetermined range, wherein:

- the initial quantity of insulin determined by the insulin determination module is the entry of the first iteration;
- the entry of each next iteration is the adjusted quantity of insulin determined at the respective previous iteration,
- the quantity of insulin to be injected is the quantity of insulin considered at the current iteration for which the predicted glycemia is within the predetermined range.

According to an embodiment, at each iteration, the quantity of insulin is adjusted by dividing it/multiplying with a number greater than 1. In an embodiment, the number used to divide/multiply the quantity of insulin is the same at each iteration. According to this embodiment, the quantity of insulin to be injected may be obtained after a few iterations only.

According to another embodiment, the number used to divide/multiply the quantity of insulin changes at each iteration. For example, when the quantity of insulin is reduced, since the quantity of insulin diminishes at each iteration, the number used to divide the quantity of insulin can also diminish at each iteration, to avoid any big gap between two consecutive quantities of insulin. The same may apply when the initial quantity is multiplied at each iteration. Consequently, a very accurate quantity of insulin to be injected can be obtained.

According to another embodiment, at each iteration the considered quantity of insulin is reduced/increased by deducting/adding a quantity of insulin. Said amount to be deducted/added can change at each iteration. For example, the amount to be deduced/added can be computed at each iteration as explained above.

According to another embodiment, the quantity of insulin can be adjusted until it reaches zero. In other words, it is determined that there is no need to inject insulin.

According to an embodiment, at each iteration, a planned sequence of insulin recommendation is optimally readjusted so that each value of the planned sequence produces a simulated glycemia trajectory within the predetermined range. For example, a Levenberg-Marquardt or any other nonlinear least squares-like algorithms can be employed to solve the constrained optimization problem consisting in approaching one of the upper and/or lower range while satisfying a glycemia trajectory within the predetermined range as well as satisfying constraints on insulin delivery.

According to an embodiment, the safety module is further configured to iteratively determine the quantity of insulin to be injected until the predicted value of glycemia is within the predetermined range, wherein:

- the entry of each iteration is the initial quantity of insulin determined by the insulin determination, wherein
- at each iteration, the safety module is configured to adjust the initial quantity of insulin by more reducing/increasing said initial quantity of insulin than at the previous iteration, and wherein
- the quantity of insulin to be injected is the quantity of insulin considered at the current iteration for which the predicted glycemia is within the predetermined range.

According to an embodiment, at each iteration, the quantity of insulin is adjusted by dividing/multiplying it with a number greater than 1. The number used to divide/multiply the quantity of insulin changes at each iteration. For example, since the entry of each iteration is the same quantity of insulin, i.e. the initial quantity of insulin determined by the insulin determination module, the number used to divide/multiply the quantity of insulin may increase/decrease at each iteration.

According to another embodiment, at each iteration the considered quantity of insulin is adjusted by deducting/adding a quantity of insulin. Said amount to be deducted/added may increase/decrease at each iteration since the entry of each iteration is the same quantity of insulin.

The reduced quantity of insulin may be equal to zero at the end of the iterations.

Both previous configurations of the iterative safety module then allows to, first, ensure even more the safety of the quantity of insulin to be injected since it verifies at each iteration that the reduced/increased quantity of insulin determined at the previous iteration will allow to have a future value of glycemia within the predetermined range if the reduced/increased quantity of insulin was injected and if a potential event will occur.

The predetermined range may be defined by a lower threshold indicating a risk of hypoglycemia and an upper threshold indicating a risk of hyperglycemia. Then, instead of only reducing/increasing the initial quantity of insulin and consider that the reduced/added quantity of insulin will avoid any risk of hypoglycemia/hyperglycemia in the future, in the case where the potential event would actually occur, the iterative loop performed by the safety module allows to ensure that the reduced/increased amount will effectively avoid any risk of hypoglycemia/hyperglycemia in the future.

Second, since the hypoglycemia is to be avoided absolutely, without the iterative configuration of the safety module, the reduced quantity of insulin could be too low, i.e. too much reduced. Indeed, without verifying if the reduced quantity of insulin will avoid the risk of hypoglycemia, the quantity of insulin will be significantly reduced, whereas by looking iteration after iteration, the quantity of insulin could be reduced little bit by little bit, ensuring to have the exact quantity of insulin that will avoid any hypoglycemia.

Finally, even if, to avoid any hypoglycemia if the potential event effectively occurs, the quantity of insulin to be injected needs to be zero, the iterative configuration of the safety module will be able to determine that the quantity of insulin to be injected is zero, after several iterations.

According to an embodiment, the safety module is further configured to stop the iterations when the quantity of insulin of the current iteration is equal to zero.

It allows to avoid a perpetual loop.

According to an embodiment, the insulin determination module is configured to determine the initial quantity of insulin by implementing a model chosen between:

- A PID controller,
- A deep learning model,
- A predictive control model,
- LQR/LQG controller,
- H∞ robust controller,
- Sliding-mode based controller,
- Adaptive controller,
- Reinforcement Learning-based controller.

Several models can be used in combination or not to determine the initial quantity of insulin.

According to an embodiment, the safety module is configured to determine the predicted glycemia according to a sensitivity to insulin factor.

It then allows to take into consideration the natural changes of the glycemia behaviour during the day. Such insulin sensitivity (IS) variations can be estimated by use of a Kalman filter providing IS estimates by coupling CGM sensor measurements with an insulin-to-glycemia state space model containing an IS state variable to be estimated. An iterative prediction/update procedure allows to first predict the IS state from the model while the update step aims at correcting the model's uncertainties by comparing the model's theoretical glycemia output to the measured glycemia and reinjecting the error to the model's raw estimate multiplied by a time-varying Kalman gain which is based on the a posteriori estimate covariance matrix, reflecting the accuracy of the previous state estimate. Such a method is described in the scientific paper Estimating insulin sensitivity after exercise using an unscented Kalman Filter, Deichman et al, IFAC PapersOnLine 54-15 (2021) 472-477.

According to an embodiment, the safety module is configured to determine the predicted glycemia according to a parameter indicating the importance of the glycemia impact of the potential event.

In an embodiment, the parameter is used in the prediction of the predicted glycemia and more precisely is directly linked to the impact that the potential event can have on glycemia. The parameter can take a value from 0.1 to 10, where 0.1 represents a situation having the least impact on glycemia, where the value 1 represents a normal situation with no impact on the glycemia, and 10 represents the worst situation with the greatest impact on glycemia.

A particular value of the parameter can be associated with each type of potential events. In an embodiment, the parameter is fixed. In another embodiment, the parameter is a variable that changes in function of contextual data. For example, it can change in function of time, for instance in the period between midnight and 3 a.m, or as a function of the glycemia value.

According to an embodiment, the potential event includes:

- A physical activity,
- An undeclared injection of a bolus of insulin,
- A change in insulin sensitivity,
- A menstrual cycle,
- An inaccurate value of current glycemia,
- An untaken meal,
- Unannounced meal,
- Stress.

Hence, all types of events having an impact on glycemia, i.e. leading to a decrease or increase of glycemia, are taken into account.

According to an embodiment, the quantity of insulin to be injected is a basal.

Such a configuration allows to take into account delivery systems unable to deliver insulin boluses and only able to deliver basal insulin, or systems able to deliver insulin boluses and basal.

According to an embodiment, the model implemented by the insulin determination module to determine the initial quantity of insulin is different and distinct from the model implemented by the safety module to determine the quantity of insulin to be injected.

Such a configuration allows to use interpretable models instead of uninterpretable models such as blackbox models for safety checks.

The present disclosure also relates to a medical system for regulating a glycemia of a person including:

- A system according to the disclosure,
- A medical device comprising:

    *A dispenser of insulin configured to inject to the person the quantity of insulin to be injected, said quantity of insulin to be injected being determined by the safety module.

**BRIEF DESCRIPTION OF THE DRAWINGS**

Embodiments of the invention will be described below with reference to the drawings, described briefly below:

[Fig. 1] schematically shows a medical system according to one embodiment of the invention.
[Fig. 2] represents a diagram illustrating a data processing unit according to one embodiment.
[Fig. 3] represents some steps implemented by the system according to an embodiment.
[Fig. 4] represents some steps implemented by the system according to another embodiment.
[Fig. 5A] represents the functioning of an existing system,
[Fig. 5B] represents the functioning of an existing system,
[Fig. 5C] represents the functioning of an existing system,
[Fig. 6A] represents the functioning of the presently disclosed system,
[Fig. 6B] represents the functioning of presently disclosed system,
[Fig. 6C] represents the functioning of presently disclosed system.

In the drawings, identical references designate identical or similar objects.

**DETAILED DESCRIPTION OF THE INVENTION**

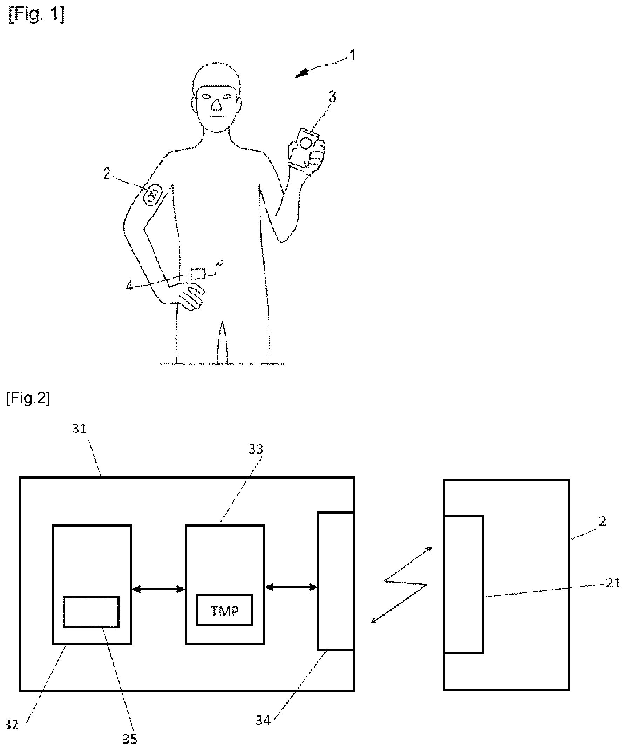

Figure 1 shows a medical system 1. The medical system 1 is advantageously used in the field of diabetes.

The medical system 1 typically includes a data acquisition system 2, a data processing unit 3 and a drug dispensing system 4, notably an insulin dispensing system 4.

The medical system 1 is a computerized medical system. In particular, the data processing unit 3 is a computerized data processing unit. Typically, the data acquisition system 2 includes a computerized data acquisition system. The insulin dispensing system 4 may be a computerized insulin dispensing system, including a processor which performs a process based on input parameters.

Any computerized system may include a processing subsystem, a storage subsystem, a user interface, a communication subsystem and a power subsystem. A computerized system may also include other components such as power battery, connectors and so on (not explicitly shown).

A storage subsystem can store data. In some embodiments, a storage subsystem can also store one or more computer programs to be executed by a processing system or a processing subsystem.

The medical system 1 may also include a user interface (not shown). The patient may be able to enter some data through the user interface, as will be described in more details below. The user interface may also be configured to deliver outputs to the patient, such as notifications.

Examples of user interface may include a screen, speakers, etc.

The data acquisition system 2 of the medical system 1 is adapted to measure a parameter of the user such as time-based data. The data acquisition system 2 may be a Continuous Glucose Monitoring (CGM) sensor. In particular, the data acquisition system 2 might be wearable by a patient. In addition, the data acquisition system 2 is adapted to repeatedly measure time-based data of the patient. In an example, the measure is made at each time step, the time step being between 1 minute and 15 minutes, preferably every 5 minutes.

Time is associated with the measured data by means of a clock of the data acquisition system 2. Such a data acquisition system may be called a continuous data acquisition system. The data acquisition system 2 may be adapted to perform in vivo measurements.

The time-based data may be a past and/or a current value of glycemia.

The data acquisition system 2 includes a data communication module 21 adapted to communicate data to the data processing unit 3. The data communication module 21 might be adapted to communicate wirelessly, for example using a short-range radio communication corresponding to the Bluetooth® standard or BLE standard.

The data processing unit 3 of the medical system 1 may include a computerized system 31 for communicating treatment information to a user. Said computerized system 31, as shown on figure 2, includes a processor 32 and a memory 33. The data processing unit 3 includes a data communication module 34 adapted to communicate data, i.e. to receive and transmit data. The data communication module 34 might be adapted to communicate wirelessly, for example using a short-range radio communication corresponding to the Bluetooth® standard. For example, the data communication module 34 of the terminal 3 and the data communication module 21 of the data acquisition system 2 are compatible with one another, to form a communication channel, by which the data processing unit 3 and the data acquisition system 2 communicate with one another. In particular, time-based data is communicated from the sensing unit 2 to the data processing unit 3. The time-based data may be stored in the memory 22.

As seen on figure 1, the medical system 1 further includes an insulin dispensing system 4.

The insulin dispensing system 4 includes a container 41 configured to contain insulin. Typically, the container 41 is able to contain a plurality of insulin doses, so as to be used a plurality of times. The insulin dispensing device 4 further includes a dispensing unit 42 adapted to dispense drug to the user. It for example includes a needle to be inserted into the body of the user, and a plunger 43 which can be pushed to expel insulin from the container 41.

In an embodiment, the data acquisition system 2, the data processing unit 3 and the insulin dispensing system 4 may communicate to each other through wireless communication, such as the Bluetooth standard. In another embodiment, they communicate with each other through wired connections.

In an embodiment, the data acquisition system 2, the data processing unit 3, the insulin dispensing system 4 and the user interface may communicate to each other through wireless communication, such as the Bluetooth standard. In another embodiment, they communicate with each other through wired connections.

In an embodiment, the data processing unit 3 could be integrated within the same unit as the data acquisition system 2 in a medical device, wearable by a patient.

In a variant embodiment, the data processing unit 3 could be integrated within the same unit as the insulin dispensing device 4, in a medical device wearable by a patient.

In yet a variant embodiment, the data acquisition system 2 could be integrated within the same unit as the insulin dispensing device 4. Thus, according to one embodiment, the data acquisition system 2, the processing system 3 and the insulin dispensing device 4 may be integrated within the same medical device wearable by a patient.

In a further embodiment, the data acquisition system 2, the data processing unit 3, the insulin dispensing system 4 and the user interface may be integrated within the same medical device wearable by a patient.

Figure 2 more specifically shows an example of the data processing unit.

The data processing unit 3 includes a computerized system 31 including a processor 32 and a memory 33 storing data. In particular, the memory stores time-based data, acquired at least in part by the data acquisition system 2. The time-based data acquired by the data acquisition system 2 are transmitted to the memory 33 of the computerized system 31, via the data communication interface 34.

The memory 33 may also store patient-related data and other possible data, as meal-related data. Such data may be transmitted by the patient, via the user interface (not shown).

The data processing system 3 is configured to ensure the safety of a quantity of insulin to be injected.

The quantity of insulin may be a bolus, a basal, a sequence of boluses and/or a sequence of basals.

To this aim, the processor 32 of the data processing unit 3 may implement a glycemia determination module 321, an insulin determination module 322 and a safety module 323.

The processor 32 may be operating data processing according, at least in part, to an artificial-intelligence based scheme, such that at least one module among the glycemia determination module 321, the insulin determination module 322 and the safety module 323 is able to implement a model working according to an artificial-intelligence, as will be explained in more detail below.

The glycemia determination module 321, the insulin determination module 322 and the safety module 323 may be able to communicate with one another.

The glycemia determination module 321 may be able to implement a model configured to determine a value of glycemia of the person. The model implemented by the glycemia determination module 321 may be stored in the memory 33.

In an embodiment, the model may be a Predictive Low Glucose Suspend (PLGS) model that determines a value of glycemia of the patient at a current instant t. The value of glycemia may be a past, a current and/or a future value of glycemia, and/or an estimated glycemic trend, i.e. a slope representative of the glycemia during an amount of time in the past, the present and/or the future.

The value of glycemia may be determined based on the time-based data and possibly also on the patient-related data. Other models able to determine a value of glycemia at an instant may be implemented.

The glycemia determination module 321 may transmit the determined value of glycemia to the insulin determination module 322.

The insulin determination module 322 may be able to implement a model configured to determine an initial quantity of insulin based, at least, on the value of glycemia determined by the glycemia determination module 321. The model implemented by the insulin determination module 322 may be stored in the memory 33.

The insulin determination module 322 may also determine the initial quantity of insulin based on the Insulin-on-board value.

In an embodiment, the insulin determination module 322 implement a MPC model, a PID controller, a Deep Learning model, a predictive control model, a LQR/LQG controller, a H∞ robust controller, a sliding-mode based model, an adaptive controller and/or a reinforcement learning-based controller, or any other model able to determine an insulin quantity.

The insulin determination module 322 may transmit the initial quantity of insulin to the safety module 323.

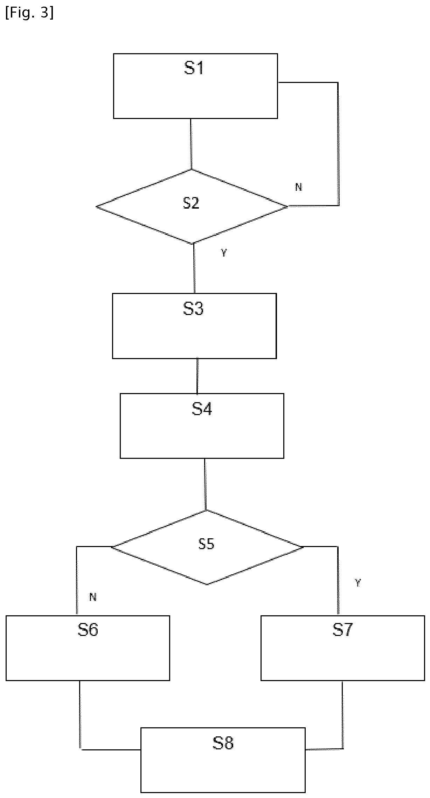

The safety module 323 may be able to implement a model configured to ensure the safety of a quantity of insulin to be injected at the current instant, as will be explained in more details below, with reference to figures 3 to 6c.

Figure 3 illustrates some steps implemented by the computerized program system during its functioning.

At step S1, the glycemia determination module 321 determines the value of glycemia, as explained above.

Optionally, step S2 is implemented, during which the glycemia determination module 321 determines if the value of glycemia obtained at step S1 satisfies a first criterion.

According to an embodiment, the first criterion is satisfied if the determined glycemia satisfies a criterion. The first criterion may correspond to a hypoglycemia risk. If the determined glycemia satisfies the first criterion, step 3 is implemented. Otherwise, step S1 is implemented again until the determined glycemia satisfies the first criterion.

Step S2 may be useful since if there is a risk of hypoglycemia, it is unlikely that the patient may need a dose of insulin. Hence, step S2 is a first safety barrier to ensure that insulin will not be delivered if there is a risk of hypoglycemia. Moreover, step S2 allows to avoid any superfluous calculation implemented in the next steps.

As stated above, step S2 is optional and step S3 could be implemented after S1 without implementing step S2.

At step S3, the insulin determination module 322 receives the glycemia value determined at step S1 and determines an initial quantity of insulin based on at least said glycemia value. The insulin determination module 322 may furthermore determine the initial quantity of insulin based on the Insulin-On-Board.

At step S4, the safety module 323 receives the initial quantity of insulin I, and performs a testing of the initial quantity of insulin I.

To do so, the safety module 323 simulates an occurrence of a potential event, the potential event having an impact on glycemia.

The potential event may be occurring at the current instant, said event having a current and/or future impact on glycemia, or at a future instant, impacting the future glycemia.

Having an impact on glycemia means that the potential event may provoke an increase or a decrease of the glycemia, respectively leading to a hypoglycemia or a hyperglycemia state.

Moreover, the potential event is not a predicted event, not an event inputted by the person, not an event determined after its occurrence, and not a consequence of an event. Indeed, this could still imply a risk for the person.

Indeed, if the safety module 323 was working based on a predicted event or on an event determined after its occurrence, the prediction or determination may be wrong, such that the event could be wrongly taken into account by the safety module 323, leading to a risk for the person. Regarding the input of a future event by the person, the person could forget or could input something wrong or may input an action that they would not perform afterwards. Then, if the safety module 323 was only relying on the input of the person, the risk may be too important, and the safety would not be completely ensured. Finally, the consequence of an event is not an event properly speaking. For example, a postprandial hypoglycemia state is not an event but the consequence of an event: the postprandial hypoglycemia may occur after a meal, where the meal is the event and the postprandial hypoglycemia the consequence.

In other words, at step S4, the safety module 323, by simulating the occurrence of a potential event, takes into account a worst case scenario and predicts the glycemia of the person taking into account the worst case scenario.

Several techniques may be employed by the safety module 323 to simulate the occurrence of the potential event and to determine the future glycemia at the future instant t+1.

In a first embodiment, the safety module 323 computes the insulin activity:

$$I[n] = X[k] * kernel(n)$$

With I[n] the insulin activity, X[k] the effect of the initial quantity of insulin and kernel(n) a function modelling the impulse response of the insulin dynamics.

Then, the safety module 323 computes the blood glucose impact:

$$BGI[n] = I[n] * ISF * dt * \gamma,$$

With BGI[n] the blood glucose impact or, in other words, the model-based impact of insulin on glycemia, I[n] the insulin activity, ISF the sensitivity to insulin factor, dt represents time, and $\gamma$ is a parameter computed from data and represents the potential event.

$\gamma$ may take a value from 0.1 to 10, where 0.1 may represent an event having the least impact on glycemia, 1 may represent a normal situation with no occurrence of an event and 10 may represent the occurrence of an event having the worst impact on glycemia. In other words, by forcing the value of $\gamma$ to 0.1, the effect of insulin on the person is reduced on purpose to avoid a hyperglycemia state and by forcing the value of $\gamma$ to 10, the effect of insulin on the person is increased on purpose to avoid a hypoglycemia state.

As can be seen from the calculation of BGI[n], the impact of insulin is increased or decreased by using the parameter $\gamma$.

Then, the safety module 323 determines the future glycemia according to:

$$predG[n+1] = predG[n] + \beta[n] * \Delta G[k] + (1- \beta[n]) * BGI[n],$$

with predG[n+1] the future glycemia at the instant t+1, predG[n] the glycemia at the current instant, $\Delta G[k]$ the glycemia variation between the glycemia at the current instant and at a past instant t-1, BGI[n] the blood glucose impact and $\beta[n]$ a weighting coefficient balancing between the deviation of the glycemia variation $\Delta G[k]$ and the deviation caused by the blood glucose impact BGI[n]. $\beta[n]$ may vary according to how far in the future the prediction is made. If $\beta[n]$ is equal to or close to 1, more confidence is accorded to the glycemia variation $\Delta G[k]$ as the trend of the glycemia is trustworthy on short term, while if $\beta[n]$ is equal to or close to 0, more confidence is accorded to the blood glucose impact BGI[n] as the insulin impact is expected to be dominant as time evolves.

$\beta[n]$ may be calculated as (1- min(1, (n-k)*dt / $\Delta$T)), where $\Delta$T controls the balance of the weighting coefficient and is usually set to 60 min.

The value of the parameter $\gamma$ may help to consider the following potential events:

A physical activity,
An undeclared injection of a bolus of insulin,
A change in insulin sensitivity,
A menstrual cycle,
An inaccurate value of current glycemia,
An untaken meal,
Stress, and/or
Unannounced meal.

At step S5, the safety module 323 determines if the predicted glycemia is within a predetermined range. In an embodiment, the lower range may correspond to a hypoglycemia state and the upper range may correspond to a hyperglycemia state.

The predicted glycemia corresponds to a value of glycemia for a future instant if the potential event had occurred and if the initial quantity of insulin had been injected at the current instant.

If the predicted glycemia is superior to the upper range, the safety module 323 is configured to adjust the initial quantity of insulin by increasing it at step S6. In an embodiment, the initial quantity of insulin will be increased by multiplying it with a factor superior to 1. In another embodiment, the initial quantity of insulin will be increased by adding an amount of insulin.

If the predicted glycemia is inferior to the lower range, the safety module 323 is configured to adjust the initial quantity of insulin by reducing it at step S6. In an embodiment, the initial quantity of insulin will be reduced by dividing it with a factor superior to 1. In another embodiment, the initial quantity of insulin will be reduced by subtracting an amount of insulin. The adjusted quantity of insulin is the quantity of insulin to be injected.

The adjusted quantity of insulin becomes the quantity of insulin to be injected.

As stated above, the quantity of insulin to be injected may be a bolus and/or a basal of insulin.

If the predicted glycemia is below the upper range and above the lower range, the initial quantity of insulin is kept and becomes the quantity of insulin to be injected at step S7.

At step S8, the quantity of insulin to be injected is sent to the insulin dispenser.

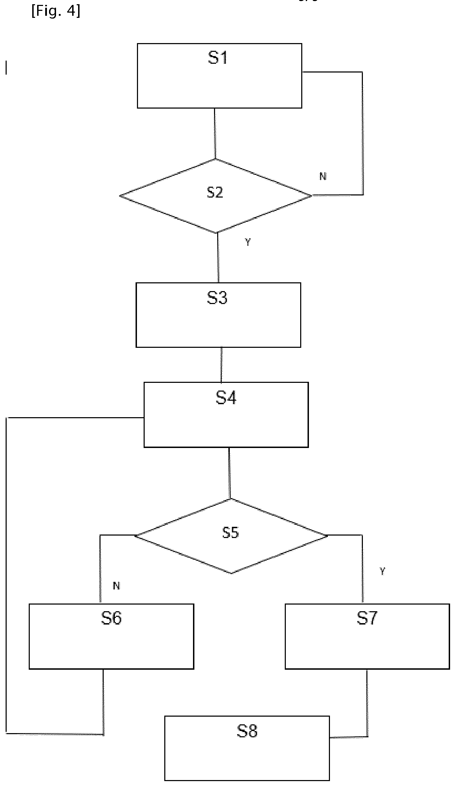

According to another embodiment illustrated on figure 4, steps S4 to S6 described with reference to figure 3 can be performed iteratively by the safety module 323 until the adjusted quantity of insulin allows to have a predicted value of glycemia within the predetermined range, i.e. below the upper range and above the lower range, or until the adjusted quantity of insulin is equal to zero.

The entry for the first iteration is the initial quantity of insulin and the entry for each of the next iterations is the adjusted quantity of insulin obtained at the respective previous iteration.

More precisely, at S5, the safety module 323 determines if the predicted glycemia is within a predetermined range or if the adjusted quantity of insulin is equal to zero. As stated above, the predicted glycemia corresponds to a value of glycemia determined for a future instant if the potential event had occurred and if the initial quantity of insulin had been injected at the current instant.

If the predicted glycemia is within the predetermined range, the system computes step S7.

If the predicted glycemia is not within the predetermined range, the initial quantity of insulin is adjusted at step S6.

Then, at each future iteration, the adjusted quantity of insulin obtained as the respective previous step is used at step S4 to test it as explained with reference to figure 3.

The iterations stop when the adjusted quantity of insulin allows to have a predicted value of glycemia within the predetermined range and/or when the adjusted quantity of insulin is equal to zero. Said adjusted quantity of insulin becomes the quantity of insulin to be injected (step S7) and is sent to the insulin dispenser (step S8).

This embodiment allows to have a more accurate adjusted quantity of insulin since the initial quantity of insulin is adjusted little by little at each iteration.

According to an embodiment, at each iteration, the quantity of insulin is adjusted by dividing it/multiplying it with a number greater than 1. In an embodiment, the number used to divide/multiply the quantity of insulin is the same at each iteration. According to this embodiment, the quantity of insulin to be injected may be obtained after a few iterations only.

According to another embodiment, the number used to divide/multiply the quantity of insulin changes at each iteration. For example, when the quantity of insulin is reduced, since the quantity of insulin diminishes at each iteration, the number used to divide the quantity of insulin can also diminish at each iteration, to avoid any big gap between two consecutive quantities of insulin. The same may apply when the initial quantity is multiplied at each iteration. Consequently, a very accurate quantity of insulin to be injected can be obtained.

According to another embodiment, at each iteration the considered quantity of insulin is reduced/increased by deducting/adding a quantity of insulin. Said amount to be deducted/added can change at each iteration. For example, the amount to be deduced/added can be computed at each iteration as explained above.

According to another embodiment, the amount to be deducted/added is the same at each iteration.

According to another embodiment, the quantity of insulin can be adjusted until it reaches zero. In other words, until it is determined that there is no need to inject insulin.

According to an embodiment, at each iteration, a planned sequence of insulin recommendation is optimally readjusted so that each value of the planned sequence produces a simulated glycemia trajectory within the predetermined range. For example, a Levenberg-Marquardt or any other nonlinear least squares-like algorithms can be employed to solve the constrained optimization problem consisting in approaching one of the predetermined glycemia thresholds while satisfying a glycemia trajectory within the predetermined range as well as satisfying constraints on insulin delivery.

In yet another embodiment, the entry of each iteration is always the initial quantity of insulin and, at each next iterations, the initial quantity of insulin is more and more reduced/increased at step S6, until the adjusted quantity of insulin allows to have a predicted glycemia within the range or until the adjusted quantity of insulin is equal to zero.

In another embodiment, the initial amount of insulin U may be determined as:

$U = kp \, (gly[t] + Td \, (gly[t] - gly[t - 1])) - IOB$, where Gly is the glycemia, IOB is the insulin on board computed from insulin delivered in the previous 8 hours.

Then, the adjusted amount of insulin U' may be determined as:

$U' = kp \, (gly[t] + Td \, (gly[t] - gly[t - 1])) - IOB - Insulin\_risk$, where Insulin_risk is defined to represent a quantity of insulin to consider a safety margin in a worst case scenario.

The worst case scenario corresponds to the occurrence of a potential event having an impact on the glycemia.

For example, such a potential event may be a bolus injection. In this case, Insulin_risk may take the value of the potential bolus to take it into account even if it does not happen.

In another example, the potential event may be a physical activity PA. Insulin_Risk may then represent the glycemia consumed by the muscles, or the increased Insulin sensitivity factor.

More precisely, in a "normal" case, with no occurrence of a physical activity, the insulin will decrease according to a factor named deltagly, but in case of a physical activity, the insulin will decrease according to a factor named deltagly_pa, with deltagly_pa > deltagly. Insulin_risk may then be computed as a function of deltagly and deltagly_pa.

Supposing that the value of glycemia Gly(s) obtained at step S1 is used to determine the initial quantity of insulin to go to a smaller value of glycemia Gly(e) at t+1. In normal condition, i.e. with no occurrence of an event having an impact on glycemia, Gly(e) = Gly(s) - Inormal * ISF, with Inormal the initial quantity of insulin and ISF the Insulin sensitivity factor.

But, in case of a physical activity, the quantity of insulin to inject Ipa to reach Gly(e) is equal to (Gly(s) - Gly(e))/ISFpa, where ISFpa is the insulin sensitivity factor in case of a physical activity.

Then, Ipa = Inormal*ISF/ISFpa, and

$$\text{Insulin\_risk} = \text{Inormal} - \text{Ipa} \quad (1 - \text{ISF/ISFpa}).$$

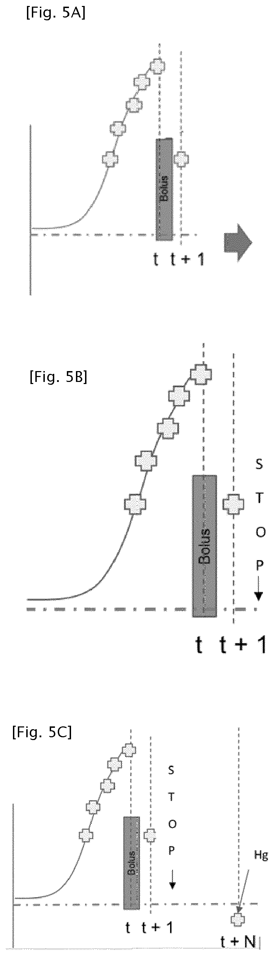

Figures 5A to 6C illustrate the functioning of the system and the difference with existing systems. On these figures, the slope represents the behaviour of the glycemia, where the crosses illustrate values of glycemia determined at instants of time until a current instant t at which an amount of insulin is to be injected to the person. The horizontal dotted line represents the lower threshold of the predetermined range, at which a risk of hypoglycemia occurs.

These figures illustrate the case where there is a risk of hypoglycemia but the same applies in the case of a risk of hyperglycemia. Furthermore, the amount of insulin is represented as a bolus in this non-limitative example.

Figures 5A to 5C illustrate the functioning of an existing system.

On figure 5A, the values of glycemia obtained in the past form an exponentially increasing slope, meaning that the value of glycemia may be too high at some point. The existing system then decides to inject at instant t a bolus. At instant t+1, the glycemia of the person then drops almost about half of its value at instant t because of the bolus injected.

On figure 5B, the existing system determines that the glycemia value is too high and that the direction of the slope changed to decrease exponentially. This may occur, first, because of the too important bolus injected at instant t, but also because the person could have performed a physical activity or other activity having a negative impact on insulin. The system then decides to stop any further injection of insulin after instant t+1.

However, as can be seen on figure 5C, this decision is taken too late since the bolus already has been injected. The person then undergoes a hypoglycemia state at t+N due to the too important bolus that has been injected. Existing systems do not allow to ensure a complete safety of an insulin injection.

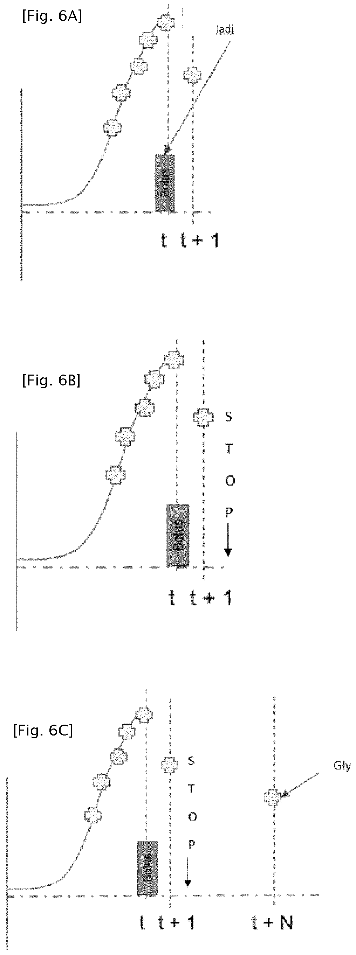

Figures 6A to 6C illustrate the functioning of the herein described system.

On figure 6A, the values of glycemia obtained in the past form an exponentially increasing slope, meaning that the value of glycemia may be too high at some point. However, at instant t, instead of performing an injection of the same bolus amount as the one on figure 5A, the system performs the testing of said amount (i.e the initial quantity of insulin) in regards with a potential event that could occur having an impact on glycemia. At instant t, the amount of insulin is then adjusted to a smaller bolus, which is then injected after determining that the future glycemia will be above the lower threshold with the injection of the adjusted amount, even if the potential event will effectively occur.

On figure 6B, it is visible that the glycemia value at instant t+1 is greater than the glycemia value at the same instant on figure 5B, due to the adjustment of the quantity of insulin. At the future instant, the system may determine that there is no need for a further injection and then decides to stop the injection of insulin.

At instant t+N (figure 6C), the glycemia of the person is then above the lower threshold, with no risk of hypoglycemia.

It can be seen from these figures that it is really efficient to simulate the worst case scenario before the injection of insulin at instant t, to avoid any risk for the person.

While exemplary embodiment of the invention has been described with reference to two main embodiments, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

**Claims**

1.  System for ensuring the safety of a quantity of insulin to be injected, said system comprising:

    - an insulin determination module (321) (322), said insulin determination module (321) (322) being configured to determine an initial quantity of insulin at a current instant, said initial quantity of insulin being determined at least based on:

        *a value of glycemia; and/or
        *an Insulin On Board (IOB) value;

    - a safety module (323), said safety module (323) configured to determine a quantity of insulin to be injected by:

        *performing a testing of the initial quantity of insulin by

            °simulating an occurrence of a potential event, wherein said potential event presents an impact on glycemia,
            °determining a predicted value of glycemia corresponding to a value of glycemia determined for a future instant if the potential event had occurred and if the initial quantity of insulin had been injected at the current instant,

        *performing a determination of whether the predicted value of glycemia is comprised within a predetermined range and

            °if no, adjust the initial quantity of insulin to obtain a adjusted quantity of insulin, said adjusted quantity of insulin being the quantity of insulin to be injected,
            °if yes, keep the initial quantity of insulin, said initial quantity of insulin being the quantity of insulin to be injected.

2.  System according to claim 1, further comprising a glycemia determination module, wherein:

    - said insulin determination module (321) is configured to determine the value of glycemia, and wherein
    - the insulin determination module (321) (322) is configured to determine the initial quantity of insulin if the determined value of glycemia satisfies a criterion.

3.  System according to any of claims 1 or 2, wherein the safety module (323) is configured to adjust the initial quantity of insulin by dividing or multiplying the initial quantity of insulin with a number greater than 1.

4.  System according to any of claims 1 or 2, wherein the safety module (323) is configured to adjust the initial quantity of insulin by deducting a quantity of insulin from said initial quantity of insulin or by adding a quantity of insulin to said initial quantity of insulin.

5.  System according to any of claims 1 to 4, wherein the safety module (323) is configured to adjust the initial quantity of insulin by forcing the value of said initial quantity of insulin to 0.

6.  System according to any of claims 1 to 5, wherein the safety module (323) is further configured to iteratively adjust the initial quantity of insulin, to obtain the quantity of insulin to be injected, until the predicted value of glycemia is within the predetermined range, wherein:

    - the initial quantity of insulin determined by the insulin determination module (321) (322) is the entry of the first iteration;
    - the entry of each next iteration is the adjusted quantity of insulin determined at the respective previous iteration,
    - the quantity of insulin to be injected is the quantity of insulin considered at the current iteration for which the predicted glycemia is within the predetermined range.

7.  System according to any of claims 1 to 5, wherein the safety module (323) is further configured to iteratively determine the quantity of insulin to be injected until the predicted value of glycemia is within the predetermined range, wherein:

- the entry of each iteration is the initial quantity of insulin determined by the insulin determination, wherein
- at each iteration, the safety module (323) is configured to adjust the initial quantity of insulin by more reducing/increasing said initial quantity of insulin than at the previous iteration, and wherein
- the quantity of insulin to be injected is the quantity of insulin considered at the current iteration for which the predicted glycemia is within the predetermined range.

8. System according to claims 6 and 7, wherein the safety module (323) is further configured to stop the iterations when the quantity of insulin of the current iteration is equal to zero.

9. System according to any of claims 1 to 8, wherein the insulin determination module (321) (322) is configured to determine the initial quantity of insulin by implementing a model chosen between:

- A PID controller,
- A deep learning model,
- A predictive control model,
- LQR/LQG controller,
- H∞ robust controller,
- Sliding-mode based controller,
- Adaptive controller,
- Reinforcement Learning-based controller.

10. System according to any of claims 1 to 9, wherein the safety module (323) is configured to determine the predicted glycemia according to a sensitivity to insulin factor.

11. System according to any of claims 1 to 10, wherein the safety module (323) is configured to determine the predicted glycemia according to a parameter indicating the importance of the glycemia impact of the potential event.

12. System according to any of claims 1 to 11, wherein the potential event includes:

- A physical activity,
- An undeclared injection of a bolus of insulin,
- A change in insulin sensitivity,
- A menstrual cycle,
- An inaccurate value of current glycemia,
- An untaken meal,
- Unannounced meal,
- Stress.

13. System according to any of claims 1 to 12, wherein the quantity of insulin to be injected is a basal.

14. System according to any of claims 1 to 13, wherein the model implemented by the insulin determination module (321) (322) to determine the initial quantity of insulin is different and distinct from the model implemented by the safety module (323) to determine the quantity of insulin to be injected.

15. Medical system (1) for regulating a glycemia of a person including:

- A system according to any of claims 1 to 14,
- A medical device comprising:

  *A dispenser of insulin (4) configured to inject to the person the quantity of insulin to be injected, said quantity of insulin to be injected being determined by the safety module (323).

[Fig. 1]

[Fig.2]

[Fig. 3]

[Fig. 4]

[Fig. 5A]

[Fig. 5B]

[Fig. 5C]

[Fig. 6A]

[Fig. 6B]

[Fig. 6C]

Europäisches
Patentamt

European
Patent Office

Office européen
des brevets

**EUROPEAN SEARCH REPORT**

Application Number

**EP 23 21 8926**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/157102 A1 (DEXCOM INC [US]) 15 August 2019 (2019-08-15) * paragraph [0302] - paragraph [0322] * * paragraph [0398] * * paragraph [0412] * * paragraph [0420] * * paragraph [0496] * * paragraph [0520] * * paragraph [0549] * * figures 1,2a,11,16,26,32b * ----- | 1-15 | INV. G16H20/17 G16H50/20 G16H50/50 A61M5/142 A61M5/172 |
| | | | TECHNICAL FIELDS SEARCHED (IPC) |
| | | | G16H A61M |

The present search report has been drawn up for all claims

| Place of search | Date of completion of the search | Examiner |
|---|---|---|
| Munich | 21 March 2024 | Gardiner, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                    

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 21 8926

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2024

| Patent document cited in search report | | Publication date | Patent family member(s) | | Publication date |
|---|---|---|---|---|---|
| WO 2019157102 | A1 | 15-08-2019 | AU | 2019217879 A1 | 23-07-2020 |
| | | | CA | 3089642 A1 | 15-08-2019 |
| | | | CN | 111655128 A | 11-09-2020 |
| | | | EP | 3749183 A1 | 16-12-2020 |
| | | | JP | 7443235 B2 | 05-03-2024 |
| | | | JP | 2021513136 A | 20-05-2021 |
| | | | JP | 2024010166 A | 23-01-2024 |
| | | | US | 2019246914 A1 | 15-08-2019 |
| | | | US | 2019246973 A1 | 15-08-2019 |
| | | | US | 2019251456 A1 | 15-08-2019 |
| | | | US | 2019252079 A1 | 15-08-2019 |
| | | | WO | 2019157102 A1 | 15-08-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10980942 B